Europäisches Patentamt

European Patent Office

Office européen des brevets

(19)

(11) **EP 0 808 903 A2**

(12) **EUROPÄISCHE PATENTANMELDUNG**

(43) Veröffentlichungstag:
26.11.1997 Patentblatt 1997/48

(21) Anmeldenummer: 97104976.2

(22) Anmeldetag: 24.03.1997

(51) Int. Cl.$^6$: **C12N 15/55**, C12N 9/18,
C12N 15/62, C12N 15/81,
C12N 1/15
// (C12N1/15, C12R1:66, 1:885)

(84) Benannte Vertragsstaaten:
BE DE FR GB IT NL

(30) Priorität: 22.05.1996 DE 19620649

(71) Anmelder: **Röhm GmbH**
64293 Darmstadt (DE)

(72) Erfinder:
• **Löffler, Fridolin, Dr. Dipl.-Chem.**
64625 Bensheim (DE)
• **Khanh, Quoc Nguyen, Dr. Dipl.-Ing.**
64385 Reichelsheim (DE)
• **Schuster, Erwin, Dr. Dipl.-Biol**
64625 Bensheim-Auerbach (DE)

• **Sprössler, Bruno, dr. Dipl.-Chem.**
64380 Rossdorf (DE)
• **Wolf, Sabine, Dr.**
64853 Otzberg (DE)
• **Thomas, Lutz, Dr. Dipl.-Ing.**
North Brunswick, NJ 08902 (US)

(74) Vertreter:
Weisert, Annekäte, Dipl.-Ing. Dr.-Ing. et al
Patentanwälte
Kraus Weisert & Partner
Thomas-Wimmer-Ring 15
80539 München (DE)

(54) **Rekombinant hergestellte Lysophospholipase aus Aspergillus**

(57) Die Erfindung betrifft eine aus Aspergillus, bevorzugt Aspergillus foetidus isolierbare rekombinante Desoxyribonukleinsäure (DNA), die dadurch gekennzeichnet ist, daß sie für eine Lysophospholipase (LPL) codiert und eine der in SEQ ID NO 1 für die reife LPL angegebene Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist. Die Erfindung betrifft weiterhin Vektoren, transformierte Wirtsorganismen sowie Verfahren zur Herstellung von LPL. Enzymprodukte zur Herstellung von Maltose-Sirup sowie entsprechend hergestellte Produkte sind ebenfalls Bestandteile der Erfindung.

EP 0 808 903 A2

SEQUENZPROTOKOLL

(1) ALLGEMEINE ANGABEN:

    (i) ANMELDER:
        (A) NAME: Roehm GmbH
        (B) STRASSE: Kirschenallee
        (C) ORT: Darmstadt
        (E) LAND: Deutschland
        (F) POSTLEITZAHL: 64293

    (ii) BEZEICHNUNG DER ERFINDUNG: Rekombinant hergestellte
        Lysophospholipase aus Aspergillus

   (iii) ANZAHL DER SEQUENZEN: 7

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 3650 Basenpaare
        (B) ART: Nucleotid
        (C) STRANGFORM: Doppelstrang
        (D) TOPOLOGIE: linear

    (ii) ART DES MOLEKÜLS: Genom-DNA

   (iii) HYPOTHETISCH: NEIN

    (iv) ANTISENSE: NEIN

    (ix) MERKMAL:
        (A) NAME/SCHLÜSSEL: intron
        (B) LAGE:1691..1743

    (ix) MERKMAL:
        (A) NAME/SCHLÜSSEL: intron
        (B) LAGE:1910..1954

    (ix) MERKMAL:
        (A) NAME/SCHLÜSSEL: intron
        (B) LAGE:2292..2344

    (ix) MERKMAL:
        (A) NAME/SCHLÜSSEL: CDS
        (B) LAGE:join(1609..1690, 1744..1909, 1955..2291, 2345
           ..2650)

(ix) MERKMAL:
    (A) NAME/SCHLÜSSEL: mat_peptide
    (B) LAGE:1690..2650

(ix) MERKMAL:
    (A) NAME/SCHLÜSSEL: sig_peptide
    (B) LAGE:1609..1689

(ix) MERKMAL:
    (A) NAME/SCHLÜSSEL: misc_feature
    (B) LAGE:2387..2425
    (D) SONSTIGE ANGABEN:/product= "Sonde KC15"

(ix) MERKMAL:
    (A) NAME/SCHLÜSSEL: misc_feature
    (B) LAGE:1563..1580
    (D) SONSTIGE ANGABEN:/product= "Oligonucleotid KC24"


(xi) SEQUENZBESCHREIBUNG: SEQ ID NO: 1:

```
CTGCAGATCA CAAGCCGGAC TGATTTTGAA CCGATTGATT GATTGAACCG CCGAAAAGTC        60

TGAAGAATCC TTCAATCCCA ACCAACACGC ACACACACAC GGTATGGATT GGAAGAAAGG       120

AAGGAAGGGA GAAAGTGGGG TTACCCAAGT AAACCGGCAA ACAGACTAAC AAACTCGCAA       180

GGAAGTTAAC TAACTAGTAA TAATATTATG AAGTGAAGGG AGTCACCTCA CCGTCTTCCC       240

GTCCCCTAAC AAACTAACTA GTTAACTACC CCGGGGTTAG TTATAGTACG GGATACTAAG       300

GCATGCACCC CGCAGCCCGG GAAGCCCGGA CGTTTGTTGT TGGTGGTTGT GACTACGGAG       360

TACTAGTCCA GTAGGGGACT CGTAAGGCAG CGGGACTAAT TAACTAGTTC GTTATTTAGG       420

CTGCCAAAGT AAATACCTAG TGCTGTGTGG GTGGTGTAAG TGCCGGGATG AAGTGAGGAG       480

TTTAGTTTTA GGTGAGTGAA TAATAACTTT CAGGTAACTA GGTAATGGAT TGACTTAGTA       540

GTTGAGTGGT TACTATCGTA GTAGACTTGG TTAGGTAATG AGAGATTTCT TTACTATAAC       600

TAGGTTATTT GTTGTATTAC TATCCTAAAA CTGTGAGAGT TCTTGGGAAG GTAAGGTAGT       660

ATTGGGGGCC GGGAATAAGG GATGAGTATT GTTTCAATAG TGAGTTGAGG TATTTTCTTT       720

TTCTATTTTC TTTTTGTCAA AGTTACTTAC TTCTTCGATA GTATGGTATT GATTATGGGC       780

ACGTGGACAT GTTCAAATTT TCGTAACATA CATAGATTGT TTGTGTGCTT TTTCCCCATC       840

TATAGTATGT ACTATGTATG TATCGTAGTC TGAATGAGTA TAAGCGCTGC AAGTGATCGG       900

CAAAGTGACA AGAAGTCACT AACTCCACTA GTTACTTGCA TGTCACGAAC CACAACCCTT       960

GACAAGCAAC AGCAACAGTA GCAGCATGTG AAGAAGTCTC AGATTTCGTA ACACCATTTC      1020

CCGTTGACTT GCTCTCCGAT TCGGTCTACT TACCTCGGCC TGTGGACATG TCATGGCCCT      1080

ATCCCCAATC TCCATCTCCA TCTCAATCTA CTGCTCATGT GCAGTACCAC GCCCTCCACG      1140
```

```
TTTCTCCGCC AGTAGATCCT TCGACACCGC CCATTCTCAG ACAACATCTA CTTTATTTAA     1200

CTTTTCGGAG CAGACTATGC TTGTCTAGGC CTGCTATAGC AGCCCGTTAA TCCCCACCGG     1260

GCTTCCGCTC CGGATGGAGA TGGGGCCAAA CTGGCAACTC CAGTTGCGCA ACGGACAACC     1320

GCCGACCCGG AACAAAGGAT GCGGATGAGG AGATACGGTG CCTGATTGCA TGGCTGGCTT     1380

CATCTGCTAT CGTGACAGTG CTCTTTGGGT GAATATTGTT GTCTGACTTA CCCCGCTTCT     1440

TGCTTTTTCC CCCCTGAGGC CCCTGATGGG GAATCGCGGT GGGTAATATG ATATGGGTAT     1500

AAAAGGGAGA TCAGAGGTGC AGTTGGATTG AGGCAGTGTG TGTGTGTGCA TTGCAGAAGC     1560

CCGTTGGTCG CAAGGTTTTG GTCGCCTCGA TTGTTTGTAT ACCGCAAG ATG TTC TCT     1617
                                                      Met Phe Ser
                                                      -27     -25
```

```
GGA CGG TTT GGA GTG CTC TTG ACA GCG CTT GCT GCG CTG GGT GCT GCC     1665
Gly Arg Phe Gly Val Leu Leu Thr Ala Leu Ala Ala Leu Gly Ala Ala
            -20             -15                     -10
```

```
GCG CCG GCA CCG CTT GCT GTG CGG A GTAGGTGTGC CCGATTTGAG             1710
Ala Pro Ala Pro Leu Ala Val Arg
            -5
```

```
GTGGTTGGAT AGCACTGATG AAGGTGTGAA TAG  GT GTC TCG ACT TCC ACG TTG     1763
                                        Ser Val Ser Thr Ser Thr Leu
                                        1                 5
```

```
GAT GAG TTG CAA TTG TTC GCG CAA TGG TCT GCC GCA GCT TAT TGC TCG     1811
Asp Glu Leu Gln Leu Phe Ala Gln Trp Ser Ala Ala Ala Tyr Cys Ser
            10                  15                  20
```

```
AAT AAT ATC GAC TCG AAA GAC TCC AAC TTG ACA TGC ACG GCC AAC GCC     1859
Asn Asn Ile Asp Ser Lys Asp Ser Asn Leu Thr Cys Thr Ala Asn Ala
        25                  30                  35
```

```
TGT CCA TCA GTC GAG GAG GCC AGT ACC ACA ATG CTG CTG GAG TTC GAC CT  1909
Cys Pro Ser Val Glu Glu Ala Ser Thr Thr Met Leu Leu Glu Phe Asp Leu
    40                  45                  50                  55
```

```
GTATGTCACT CAGATCGCAG ACATAGAGCA CAGCTAATTG AACAG G ACG AAC GAC     1964
                                                    Thr Asn Asp
```

```
TTT GGA GGC ACA GCC GGT TTC CTG GCC GCG GAC AAC ACC AAC AAG CGG     2012
Phe Gly Gly Thr Ala Gly Phe Leu Ala Ala Asp Asn Thr Asn Lys Arg
60                  65                  70                  75
```

```
CTC GTG GTC GCC TTC CGG GGA AGC AGC ACG ATT GAG AAC TGG ATT GCT     2060
Leu Val Val Ala Phe Arg Gly Ser Ser Thr Ile Glu Asn Trp Ile Ala
                80                  85                  90
```

```
AAT CTT GAC TTC ATC CTG GAA GAT AAC GAC GAC CTC TGC ACC GGC TGC     2108
Asn Leu Asp Phe Ile Leu Glu Asp Asn Asp Asp Leu Cys Thr Gly Cys
            95                  100                 105
```

16

```
AAG GTC CAT ACT GGT TTC TGG AAG GCA TGG GAG TCC GCT GCC GAC GAA      2156
Lys Val His Thr Gly Phe Trp Lys Ala Trp Glu Ser Ala Ala Asp Glu
        110                 115                 120

CTG ACG AGC AAG ATC AAG TCT GCG ATG AGC ACG TAT TCG GGC TAT ACC      2204
Leu Thr Ser Lys Ile Lys Ser Ala Met Ser Thr Tyr Ser Gly Tyr Thr
        125                 130                 135

CTA TAC TTC ACC GGG CAC AGT TTG GGC GGC GCA TTG GCT ACG CTG GGA      2252
Leu Tyr Phe Thr Gly His Ser Leu Gly Gly Ala Leu Ala Thr Leu Gly
140                 145                 150                 155

GCG ACA GTT TTG CGA AAT GAC GGG TAT AGC GTT GAG CTG GTGAGTCCTT       2301
Ala Thr Val Leu Arg Asn Asp Gly Tyr Ser Val Glu Leu
                160                 165

CACAAAGGTG ATGGAGCGAC AATCGGGTTC TGACAGTCAA TAG TAC ACC TAT GGA      2356
                                                   Tyr Thr Tyr Gly
                                                           170

TGT CCT CGA ATC GGA AAC TAT GCG CTG GCT GAG CAT ATC ACC AGT CAG      2404
Cys Pro Arg Ile Gly Asn Tyr Ala Leu Ala Glu His Ile Thr Ser Gln
        175                 180                 185

GGA TCT GGG GCC AAC TTC CGT GTT ACA CAC TTG AAC GAC ATC GTC CCC      2452
Gly Ser Gly Ala Asn Phe Arg Val Thr His Leu Asn Asp Ile Val Pro
        190                 195                 200

CGG GTG CCA CCC ATG GAC TTT GGA TTC AGT CAG CCA AGT CCG GAA TAC      2500
Arg Val Pro Pro Met Asp Phe Gly Phe Ser Gln Pro Ser Pro Glu Tyr
205                 210                 215                 220

TGG ATC ACC AGT GGC AAT GGA GCC AGT GTC ACG GCG TCG GAT ATT GAA      2548
Trp Ile Thr Ser Gly Asn Gly Ala Ser Val Thr Ala Ser Asp Ile Glu
                225                 230                 235

GTC ATC GAG GGA ATC AAT TCA ACG GCG GGA AAT GCA GGC GAA GCA ACG      2596
Val Ile Glu Gly Ile Asn Ser Thr Ala Gly Asn Ala Gly Glu Ala Thr
                240                 245                 250

GTG AGC GTT TTG GCT CAC TTG TGG TAC TTT TTC GCG ATT TCC GAG TGC      2644
Val Ser Val Leu Ala His Leu Trp Tyr Phe Phe Ala Ile Ser Glu Cys
        255                 260                 265

CTG CTA TAACTAGACC GACTGTCAGA TTAGTGGACG GGAGAAGTGT ACATAGTAAT       2700
Leu Leu
        270

TAGTATATAA TCAGAGCAAC CCAGTGGTGG TGATGGTGGT GAAAGAAGAA ACACAGTGAG    2760

ATCCCATTAC GTAGCAGATA AAGCACGTGT GGAGGCGCTG TTCCTCCACT TGCAGTTGCG    2820

GCCATCAATC ATCTTTCCTC TCCTTACTTT CGTCCACCAC AACTCCCATC CTGCCAGCTG    2880

TCGCATCCCC GGGTTGCAAC AACTATCGCC TCCGGGGCCT CCGTGGTTCT CCTATATTAT    2940

TCCATCCGAC GGCCGACGTT TCACCCTCAT CCTGCGCCGC CGCAAAATCT CCCCGAGTCG    3000
```

17

```
GTCAACTCCC TCGAACCGCC GCCCGCATCA ACCTCACCGA CCCCCGACCG TCTGCGATCG     3060

TCCAACCGTG CAGCCATGTT CCCTGCCCCG TCACGCTGAT TTGACGCTCA ATTGACTTCC     3120

GTGACATACA TACATACATC CAGCAGCTTA TGATCACAGT TTCCCCGCCT CGTGCCAGTC     3180

CCGCCGCCGC CATTGCCAGC CATGCTATTC TTCCGCGGCG GAGTTAACAG TCCGCGCCGG     3240

CGCAACCAAG AAAAAGCAAT CTTCTGCACC ATTCTCATCA TCTACGCTCT ATACTTCCTC     3300

TTCTTCGCTG GGAGCTCCGA TCCCAAACGG TCAGAGACGT CTGCTGAGGA TGCGGTGAAG     3360

GGAGGCCCGC ATCGGGACTC GCGGTCGGGG AAGGCTTCCC CGTCCGCCTC GGCTTCGCGA     3420

CAGCCCCAGG CCTTGGACAA GGACATGGTG GTGGCTAGTA TGAAGGGAGA CGATGTCTCA     3480

TGGCTGTACA CATACTTTCC TGAGTGGCAC AAAAGCATCT ACGTGGCGGA CGACAAGAAA     3540

GCGCCGTTAA CGGTGGCCTT GAATAAGGGG AGGGAATCGA TGGTGTATCT GACGTGAGTA     3600

GTCTCCTCCG TGTCTTGGCC GGTTCTGGGC GCACCAACTC ACTGCTGCAG               3650
```

(2) ANGABEN ZU SEQ ID NO: 2:

    (i) SEQUENZKENNZEICHEN:
      (A) LÄNGE: 297 Aminosäuren
      (B) ART: Aminosäure
      (D) TOPOLOGIE: linear

    (ii) ART DES MOLEKÜLS: Protein
    (xi) SEQUENZBESCHREIBUNG: SEQ ID NO: 2:

```
Met Phe Ser Gly Arg Phe Gly Val Leu Leu Thr Ala Leu Ala Ala Leu
-27     -25             -20             -15

Gly Ala Ala Ala Pro Ala Pro Leu Ala Val Arg Ser Val Ser Thr Ser
    -10                 -5                   1               5

Thr Leu Asp Glu Leu Gln Leu Phe Ala Gln Trp Ser Ala Ala Ala Tyr
                10               15                   20

Cys Ser Asn Asn Ile Asp Ser Lys Asp Ser Asn Leu Thr Cys Thr Ala
            25               30                   35

Asn Ala Cys Pro Ser Val Glu Glu Ala Ser Thr Thr Met Leu Leu Glu
        40               45                   50

Phe Asp Leu Thr Asn Asp Phe Gly Gly Thr Ala Gly Phe Leu Ala Ala
    55               60                   65

Asp Asn Thr Asn Lys Arg Leu Val Val Ala Phe Arg Gly Ser Ser Thr
    70               75                   80                ·85

Ile Glu Asn Trp Ile Ala Asn Leu Asp Phe Ile Leu Glu Asp Asn Asp
                90               95                   100
```

Asp Leu Cys Thr Gly Cys Lys Val His Thr Gly Phe Trp Lys Ala Trp
       105             110              115

Glu Ser Ala Ala Asp Glu Leu Thr Ser Lys Ile Lys Ser Ala Met Ser
       120             125            130

Thr Tyr Ser Gly Tyr Thr Leu Tyr Phe Thr Gly His Ser Leu Gly Gly
   135             140            145

Ala Leu Ala Thr Leu Gly Ala Thr Val Leu Arg Asn Asp Gly Tyr Ser
150            155          160           165

Val Glu Leu Tyr Thr Tyr Gly Cys Pro Arg Ile Gly Asn Tyr Ala Leu
       170             175           180

Ala Glu His Ile Thr Ser Gln Gly Ser Gly Ala Asn Phe Arg Val Thr
       185             190           195

His Leu Asn Asp Ile Val Pro Arg Val Pro Pro Met Asp Phe Gly Phe
       200             205           210

Ser Gln Pro Ser Pro Glu Tyr Trp Ile Thr Ser Gly Asn Gly Ala Ser
       215             220           225

Val Thr Ala Ser Asp Ile Glu Val Ile Glu Gly Ile Asn Ser Thr Ala
230            235          240           245

Gly Asn Ala Gly Glu Ala Thr Val Ser Val Leu Ala His Leu Trp Tyr
       250             255           260

Phe Phe Ala Ile Ser Glu Cys Leu Leu
       265             270

(2) ANGABEN ZU SEQ ID NO: 3:

    (i) SEQUENZKENNZEICHEN:
       (A) LÄNGE: 32 Basenpaare
       (B) ART: Nucleotid
       (C) STRANGFORM: Einzelstrang
       (D) TOPOLOGIE: linear

   (ii) ART DES MOLEKÜLS: Genom-DNA

 (iii) HYPOTHETISCH: NEIN

  (iv) ANTISENSE: NEIN


  (ix) MERKMAL:
       (A) NAME/SCHLÜSSEL: misc_feature
       (B) LAGE:18
       (D) SONSTIGE ANGABEN:/product= "N ist Inosin"

  (ix) MERKMAL:
       (A) NAME/SCHLÜSSEL: misc_feature
       (B) LAGE:24
       (D) SONSTIGE ANGABEN:/product= "N ist Inosin"

```
(ix) MERKMAL:
     (A) NAME/SCHLÜSSEL: misc_feature
     (B) LAGE:30
     (D) SONSTIGE ANGABEN:/product= "N ist Inosin"


(xi) SEQUENZBESCHREIBUNG: SEQ ID NO: 3:

TCCAAGCTTG ACGAACTNCA ACTNTTCGCN CA                          32

(2) ANGABEN ZU SEQ ID NO: 4:

  (i) SEQUENZKENNZEICHEN:
     (A) LÄNGE: 40 Basenpaare
     (B) ART: Nucleotid
     (C) STRANGFORM: Einzelstrang
     (D) TOPOLOGIE: linear

 (ii) ART DES MOLEKÜLS: Genom-DNA

(iii) HYPOTHETISCH: NEIN

 (iv) ANTISENSE: NEIN


 (ix) MERKMAL:
     (A) NAME/SCHLÜSSEL: misc_feature
     (B) LAGE:40
     (D) SONSTIGE ANGABEN:/product= "N ist A, C oder G"


 (xi) SEQUENZBESCHREIBUNG: SEQ ID NO: 4:

GACTCGAGTC GACATCGATT TTTTTTTTTT TTTTTTTTTN                  40

(2) ANGABEN ZU SEQ ID NO: 5:

  (i) SEQUENZKENNZEICHEN:
     (A) LÄNGE: 20 Basenpaare
     (B) ART: Nucleotid
     (C) STRANGFORM: Einzelstrang
     (D) TOPOLOGIE: linear

 (ii) ART DES MOLEKÜLS: Genom-DNA

(iii) HYPOTHETISCH: NEIN

 (iv) ANTISENSE: NEIN



 (xi) SEQUENZBESCHREIBUNG: SEQ ID NO: 5:

GACTCGAGTC GACATCGATT                                        20

(2) ANGABEN ZU SEQ ID NO: 6:
```

```
(i) SEQUENZKENNZEICHEN:
    (A) LÄNGE: 42 Basenpaare
    (B) ART: Nucleotid
    (C) STRANGFORM: Einzelstrang
    (D) TOPOLOGIE: linear

(ii) ART DES MOLEKÜLS: Genom-DNA

(iii) HYPOTHETISCH: NEIN

(iv) ANTISENSE: NEIN
```

```
(xi) SEQUENZBESCHREIBUNG: SEQ ID NO: 6:
```

GGAATTCACC TGCTAACCAT GTTCTCTGGA CGGTTTGGAG TG                                    42

(2) ANGABEN ZU SEQ ID NO: 7:

```
(i) SEQUENZKENNZEICHEN:
    (A) LÄNGE: 24 Basenpaare
    (B) ART: Nucleotid
    (C) STRANGFORM: Einzelstrang
    (D) TOPOLOGIE: linear

(ii) ART DES MOLEKÜLS: Genom-DNA

(iii) HYPOTHETISCH: NEIN

(iv) ANTISENSE: NEIN
```

```
(xi) SEQUENZBESCHREIBUNG: SEQ ID NO: 7:
```

CGCCAGGGTT TTCCCAGTCA CGAC                                                        24

**Beschreibung**

Die Erfindung betrifft eine rekombinante Desoxyribonukleinsäure (DNA) aus Aspergillus, bevorzugt aus <u>Aspergillus foetidus</u>, die für eine Lysophospholipase (LPL, Phospholipase B, 2-Lysophosphatidylcholin-Acylhydrolase, IUB 3.1.1.5) codiert, Vektoren, die diese DNA sowie weitere DNA-Sequenzen zur Expression des LPL-Gens enthalten, sowie mit diesen Vektoren transformierte filamentöse Pilze, die die rekombinante DNA exprimieren können. Weiterhin betrifft die Erfindung Enzymprodukte, die eine mittels der rekombinanten filamentösen Pilze hergestellte rekombinante LPL enthalten, sowie Verfahren zur Verbesserung der Filtration von Stärkehydrolysaten mittels der rekombinanten LPL.

Bei der Herstellung von Stärkehydrolysaten besteht die Schwierigkeit, die entstehende hochkonzentrierte Lösung von Glucoseoligomeren von verbleibenden Trubstoffen durch Filtration zu befreien. Diese Trubstoffe bestehen ganz oder teilweise aus Phospholipiden, die durch Phospholipide spaltende Enzyme hydrolysiert werden, wodurch der Filtrationsvorgang beschleunigt wird.

Phospholipide spaltende Enzyme, die für diese Anwendung geeignet sind, sind in der EP 0 219 269 und von Konieczny-Janda, G. und Richter, G.: Starch/Stärke (1991), <u>43</u>(8), S. 308-315 beschrieben. Das EP 0 219 269 beschreibt eine Phospholipase aus Aspergillus niger, die Xylanase und β-Glucanase in geeigneten Verhältnissen enthält und die Filtrationsleistung bei der Klärung von Maltose-Sirup von 100 auf 300 l/h • m$^2$ erhöht. Konieczny-Janda und Richter isolierten aus Glucoamylase von Aspergillus niger ein Enzym, das die Lysophospholipide der Weizenstärke partiell hydrolysiert, so daß die unlöslichen Bestandteile aggregieren und damit filtrierbar werden. Das isolierte Enzym spaltet Weizen-Lipide und synthetische Lysophospholipide.

Der vorliegenden Erfindung liegt die Aufgabe zugrunde, eine kostengünstige Lysophospholipase für die Filtration von Stärkesirup, speziell Maltose-Sirup, der durch β-Amylase-Behandlung von Maltodextrinlösungen gewonnen wurde, bereitzustellen. Ferner soll die Lysophospholipase frei von Glucoamylasen und Transglucosidasen sein. Weiterhin soll die Lysophospholipase durch einen transformierten Wirtsorganismus in großen Mengen produziert werden können. Mit dem Enzym sollen Präparate hergestellt werden können, die sich besonders gut zur Hydrolyse von Lysophospholipiden und somit zur Klärung von Stärkehydrolysaten eignen.

Erfindungsgemäß wird die Aufgabe gelöst durch eine aus Aspergillus isolierbare rekombinante Desoxyribonukleinsäure (DNA), die dadurch gekennzeichnet ist, daß sie für eine Lysophospholipase (LPL) codiert und die in SEQ ID NO 1 für die reife LPL angegebene Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist. Bevorzugt wird die DNA aus Aspergillus foetidus isoliert.

Mit dieser DNA können Vektoren, insbesondere Plasmide, hergestellt werden, mit denen Aspergillus-Stämme oder Trichoderma reesei-Stämme transformiert werden können. Aus den erhaltenen Transformanten können dann solche Stämme selektiert werden, die LPL in hohen Mengen exprimieren und sezernieren. Diese transformierten Wirtsorganismen erlauben wiederum Verfahren zur Herstellung der LPL in hohen Mengen. Aus den so gewonnenen Fermentationssäften können LPL-haltige Enzymprodukte hergestellt werden, die besonders gut zur Hydrolyse von Lysophospholipiden geeignet sind. Durch die Auswahl des Wirtsorganismus ist es möglich, die Lysophospholipase mit hoher Ausbeute und im wesentlichen frei von störenden Enzymen wie Glucoamylasen und Transglucosidasen herzustellen, so daß mit dem erfindungsgemäß hergestellten Enzymprodukt die Filtration von Maltose-Sirup in höherem Maße beschleunigt und gleichzeitig die Bildung von unerwünschten Nebenprodukten in dem geklärten Sirup verringert werden kann als dies mit konventionell hergestellten Präparaten möglich ist.

Die Erfindung wird anhand der folgenden Figuren näher erläutert.

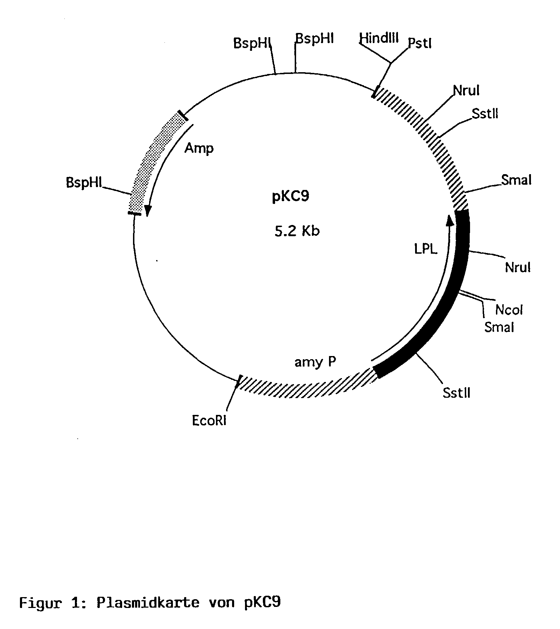

Die Figur 1 zeigt die Plasmidkarte des Vektors pKC9.

Die Figur 2 zeigt die Plasmidkarte des Vektors pKC3.

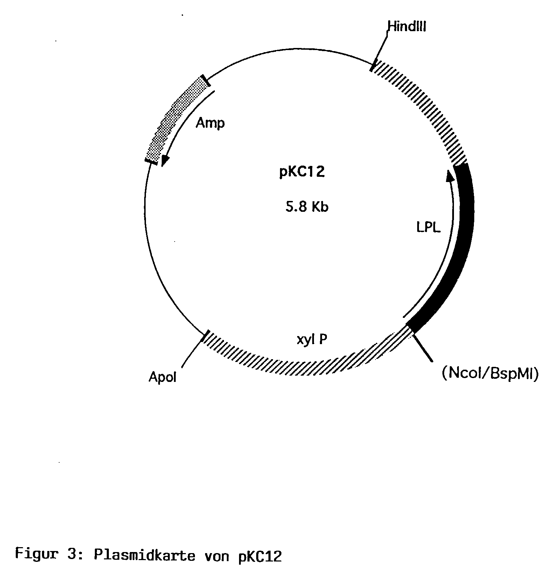

Die Figur 3 zeigt die Plasmidkarte des Vektors pKC12.

Die folgenden Mikroorganismen wurden bei der Deutschen Sammlung von Mikroorganismen und Zellkulturen (DSM), Mascheroder Weg 1B, 38124 Braunschweig, Deutschland, gemäß den Bestimmungen des Budapester Vertrages hinterlegt:

|  |  |
|---|---|
| A. foetidus RH3046: | Hinterlegungsnummer DSM 10652 |
| E. coli DH5α pKC3: | Hinterlegungsnummer DSM 10653 |
| E. coli DH5α pKC9: | Hinterlegungsnummer DSM 10654 |
| E. coli DH5α pKC12: | Hinterlegungsnummer DSM 10655 |

<u>Rekombinante DNA</u>

Die Erfindung betrifft eine rekombinante Desoxyribonukleinsäure (DNA), isolierbar aus Aspergillus foetidus, die dadurch gekennzeichnet ist, daß sie für eine Lysophospholipase (LPL) codiert und die in SEQ ID NO 1 für die reife LPL angegebene Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist.

2

Die in SEQ ID NO 1 dargestellte Sequenz entspricht dem chromosomalen LPL-Gen aus A. foetidus RH3046 mit 5'- und 3'-flankierenden Sequenzen. Unter der Nukleotidsequenz für die reife LPL wird die gemäß SEQ ID NO 1 erhaltene, für das Strukturgen der LPL ohne das Signalpeptid codierende DNA-Sequenz verstanden. Die Nukleotidsequenz für die reife LPL sind somit diejenigen Exon-Sequenzen, die für die Aminosäuren 1 (Ser) bis 270 (Leu) codieren.

Die Erfindung betrifft weiterhin eine von der Nukleotidsequenz für die reife LPL abgeleitete Nukleotidsequenz. Darunter sind Nukleotidsequenzen zu verstehen, die von der in SEQ ID NO 1 angegebenen Nukleotidsequenz abweichen, aber unter stringenten Bedingungen mit dieser DNA für die LPL hybridisieren.

Der Begriff "stringente Hybridisierungsbedingungen" ist dem Fachmann geläufig (siehe z.B. Maniatis et al., (1982), Molecular Cloning, A Laboratory Manual, Cold Spring Harbor Laboratory, New York). Stringente Hybridisierungsbedingungen sind solche, unter denen nur DNA-Moleküle mit einem hohen Homologiegrad, z.B. > 85 %, miteinander hybridisieren. Die Stringenz der Versuchsbedingungen kann z.B. durch die Hybridisierungstemperatur oder durch die Salzkonzentration der Hybridisierungslösung eingestellt werden.

Vom LPL-Strukturgen abgeleitete DNA-Sequenzen können z.B. Abweichungen in der Nukleotidsequenz ohne Abweichungen in der Aminosäuresequenz, bedingt durch die Degeneration des genetischen Codes, aufweisen. Ebenso können geringfügige Abweichungen in der Nukleotidsequenz auch zu funktionell unwesentlichen Änderungen in der Aminosäuresequenz des Enzyms führen. Weitgehend homologe Gene, die mittels der vorliegenden Erfindung aus anderen Stämmen von A. foetidus oder nah verwandten Aspergillus-Arten isoliert werden können, sind daher eingeschlossen. Die Erfindung umfaßt auch Fusionsproteine, die für die enzymatische Funktion des Proteins wesentliche Teile der Teile des LPL-Gens bzw. davon abgeleitete Nukleotidsequenzen aufweisen.

Die Sequenzbeschreibung gemäß SEQ ID NO 1 enthält weiterhin die vor der Nukleotidsequenz für die reife LPL liegende codierende Sequenz für das Signalpeptid, beginnend mit der Aminosäure 1 (Met) bis 21 (Pro). Anschließend folgt ein Propeptid mit sechs Aminosäuren, von Position 22 (Ala) bis 27 (Arg). Vor der Signalpeptidsequenz liegt die funktionelle Promotorregion des LPL-Gens (Nukleotid 1 bis 1608). Hinter dem Strukturgen befindet sich das TAA-Stopcodon sowie eine als Transkriptionsterminator funktionelle Region (Nukleotide 2654 bis 3650). Die Sequenzbeschreibung gemäß SEQ ID NO 1 gibt die gesamte Aminosäuresequenz der LPL mit dem Signalpeptid wieder.

Die erfindungsgemäße rekombinante DNA kann durch die Isolierung eines LPL-Gens aus dem Genom eines LPL-produzierenden Aspergillus-Stammes, insbesondere eines A. foetidus-Stammes, z.B. aus A. foetidus RH3046, erhalten werden. Dazu wird RNA und/oder DNA aus dem Zellmaterial des Aspergillus-Stammes gewonnen.

Für die Isolierung der RNA wird das Zellmaterial bevorzugt in einem Medium gezüchtet, in dem der Stamm möglichst viel LPL produziert, da in diesem induzierten Zellmaterial der Gehalt an LPL-spezifischer RNA höher als in nichtinduziertem Zellmaterial ist. Dadurch wird ein höherer Anteil an LPL-spezifischer cDNA bei der cDNA-Synthese erhalten, wodurch die Isolierung der LPL-cDNA erleichtert wird.

Geeignete Anzuchtmedien sind z.B. Minimalmedien für Pilze, denen Lysolecithin enthaltende Bestandteile, z.B. eine Fraktion aus Soja, zugesetzt ist. Bei maximalem LPL-Titer im Medium erfolgt die Zellernte vorteilhafterweise durch das Einfrieren des abfiltrierten Mycels in flüssigem Stickstoff.

Aus dem Zellmaterial kann die Präparation von RNA in an sich bekannter Weise erfolgen. Dazu wird das Mycel z.B. in Gegenwart von Detergentien wie SDS (Natriumdodecylsulfat) unter flüssigem Stickstoff homogenisiert und die RNA durch Phenol-, Phenol-Chloroform-Extraktionen, ggf. unter Zusatz von Isoamylalkohol, gewonnen. Die RNA wird anschließend aus der wäßrigen Phase durch Zusatz von Salzen, wie z.B. Lithiumchlorid, ausgefällt.

Aus der RNA kann in an sich bekannter Weise mittels reverser Transkriptase (RNA-abhängige DNA-Polymerase) zunächst eine komplementäre einzelsträngige DNA erzeugt werden. Dazu wird die RNA mit einer Mischung von Desoxynukleosidtriphosphaten (dATP, dCTP, dGTP und dTTP), einem Oligo(dT)-Primer, der mit dem Poly(A)-Ende der mRNA hybridisiert, und einer reversen Transkriptase, z.B. der reversen Transkriptase des Moloney-Maus-Leukämie-Virus (M-MuLV), inkubiert, die eine komplementäre einzelsträngige DNA erzeugt.

In einem zweiten Schritt wird dann zu der einselsträngigen DNA mit der DNA-Polymerase wiederum ein Komplementärstrang synthetisiert, so daß nun eine doppelsträngige cDNA vorliegt. Die Ausbeute an LPL-spezifischer cDNA kann mittels der Tag-DNA-Polymerase in an sich bekannter Weise mit der Polymerase-Kettenreaktion (PCR) um ein Vielfaches erhöht werden. Dazu wird die einzelsträngige cDNA mit einer Mischung von Desoxynukleosidtriphosphaten, einer entsprechenden Teilsequenz des Oligo(dT)-Primers für die Rückreaktion und einem für die Hinreaktion LPL-spezifischen Primer inkubiert. Der LPL-spezifische Primer wird aus der Aminosäuresequenz der LPL abgeleitet und soll theoretisch nur mit einem bestimmten Bereich der einzelsträngigen LPL-cDNA hybridisieren. Die cDNA-Synthese wird mit der Taq-DNA-Polymerase gestartet und verläuft somit nur in einem definierten Bereich, d.h. zwischen zwei Oligonukleotiden. Die PCR-Produkte werden gereinigt und in das Plasmid pUC18 insertiert. Nach der Transformation in E. coli DH5α kann die Plasmid-DNA aus den ausgewählten Kolonien präpariert und anschließend mit einer radioaktiv markierten DNA-Sonde hybridisiert werden. Die cDNA-Insertion eines positiven Klons wird sequenziert. Eine Übereinstimmung der von der Nukleotidsequenz abgeleiteten Aminosäuresequenz mit der durch Edman-Abbau bestimmten Sequenz bestätigt das gesuchte LPL-Gen.

Die gewonnene LPL-cDNA kann als Gensonde für die Southern-Hybridisierung und die genomische Klonierung der LPL benutzt werden.

Die Isolierung des chromosomalen LPL-Gens kann durch direkte Klonierung der mit Restriktionsenzymen hydrolysierten chromosomalen DNA in das Plamid pUC18 erfolgen.

Genomische DNA kann unabhängig von den Anzuchtbedingungen der Zellen aus dem Mycel gewonnen werden. Bevorzugt ist eine Anzucht des Zellmaterials in einem Vollmedium für Pilze, z.B. Sabouraud-Bouillon. Die Isolierung der DNA aus dem Zellmaterial kann in an sich bekannter Weise durch Homogenisierung des Zellmaterials in einem Puffer, z.B. 100 mM Tris-HCl, pH 7,5, 300 mM NaCl, 200 mM EDTA, und anschließende Extraktion mit Phenol, Chloroform-Isoamylalkohol erfolgen. Die DNA wird durch Zusatz von Ethanol und Natriumacetat aus der wäßrigen Phase präzipitiert. Sie wird mit einem Restriktionsenzym, z.B. BamHI, vollständig gespalten und anschließend in einem Saccharose-Gradienten nach Größe fraktioniert. Aufgrund des Ergebnisses aus der Southern-Hybridisierung werden die Fraktionen mit DNA-Fragmenten der Größe von ca. 9 bis 10 kb gesammelt. Nach der Fällung der DNA mit Ethanol wird die DNA in den Vektor pUC18 insertiert. Nach der Transformation in E. coli DH5α können die gesuchten LPL-Transformanten mittels Koloniehybridisierung identifiziert werden. Die Charakterisierung der isolierten DNA erfolgt in an sich bekannter Weise durch Restriktionsanalyse und anschließende Sequenzierung, z.B. nach der Sanger-Methode.

Durch den Vergleich der cDNA-Sequenz mit der Sequenz des chromosomalen Gens läßt sich die Lage der Exon- und Intron-Sequenzen bestimmen.

Vektoren zur Expression der LPL in Aspergillus- oder T. reesei-Stämmen

Die Erfindung betrifft weiterhin einen Vektor, enthaltend

a) DNA-Sequenzen zur Replikation des Vektors in E. coli,
b) DNA-Sequenzen zur Expression und Sekretion eines Polypeptids in einem Aspergillus-Stamm oder in einem Trichoderma reesei-Stamm, die für einen Promotor, eine Signalpeptidsequenz und optional für einen Terminator codieren,
c) eine für ein Polypeptid codierende DNA-Sequenz, die funktionell mit den DNA-Sequenzen nach b) verbunden ist,

der dadurch gekennzeichnet ist, daß die DNA-Sequenz nach c) eine Nukleotidsequenz entsprechend der in SEQ ID NO 1 für die reife LPL angegebenen Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist.

Die DNA-Sequenzen nach a) werden benötigt, um die Vektor-DNA in E. coli, z.B. E. coli DH5α, vermehren zu können. Eine derartige DNA-Sequenz kann ein Phage, z.B. der Phage EMBL3, ein Cosmid oder bevorzugterweise ein Plasmid sein. Geeignete Plasmide sind z.B. pBR322, pUC18 oder pUC19 oder ggf. Fragmente dieser Plasmide, die zumindest den Replikationsorigin und einen Selektionsmarker für E. coli enthalten.

Die Vektoren enthalten weiterhin DNA-Sequenzen nach b), die in Aspergillus-Stämmen oder in T. reesei-Stämmen zur Expression und Sekretion des Genprodukts des LPL-Gens nach c) führen. Diese DNA-Sequenzen nach b) sind mit dem LPL-Gen funktionell verbunden. Dies sind z.B. 5'- und 3'-flankierende Sequenzen, wie 5' des Gens angeordnete Promotoren, eine Signalpeptidsequenz, 3' des Gens angeordnete Terminatoren. Weitere funktionelle DNA-Sequenzen, die vorhanden sein können, sind z.B. Ribosomenbindungsstellen für die Translation, Enhancer oder "Upstream Activating Sequences" oder Polyadenylierungsfunktionen.

Signalpeptidsequenzen sind 5' unmittelbar vor dem Strukturgen liegende, für Aminosäuren codierende DNA-Sequenzen, die bei extrazellulären Proteinen vorkommen und gemeinsam mit dem Strukturgen transkribiert und translatiert werden. Bei der Sekretion des Proteins aus der Zelle werden die Signalpeptidsequenzen abgespalten, wodurch das eigentliche "reife" Protein entsteht. Eine Signalpeptidsequenz ist somit notwendig, um die Sekretion der LPL aus der Wirtszelle zu erreichen. Ebenso sind ein Promotor und ein Terminator zur Initiation der Translation bzw. zur Termination der Transkription des Gens notwendig.

Es kann sich dabei um den natürlich im Chromosom von A. foetidus liegenden Promotor, die Signalpeptidsequenz und/oder den Terminator des LPL-Gens handeln. Ebenso können die funktionellen DNA-Sequenzen von anderen Genen stammen, deren Produkte in Pilzstämmen der Gattung Aspergillus oder in T. reesei exprimiert und sezerniert werden.

Beispiele für Gene, die geeignete funktionelle DNA-Sequenzen aufweisen, sind z.B. das TAKA-Amylase A-Gen aus A. oryzae (EP 0 238 023), das Pektinesterase-Gen oder das Polygalakturonidase-Gen aus A. niger (EP 0 388 593), das Glucoamylase-Gen aus A. awamori (EP 0 215 594), das Cellobiohydrolase-Gen I (cbh 1) aus T. reesei (EP 0 244 234).

Die DNA-Sequenz nach c) enthält das LPL-Strukturgen oder eine davon abgeleitete DNA-Sequenz. Diese DNA-Sequenz kann z.B. in Form eines chromosomalen Gens mit den natürlich enthaltenen Introns oder als von der Messenger-Ribonukleinsäure (mRNA) abgeleitete cDNA ohne Introns enthalten sein.

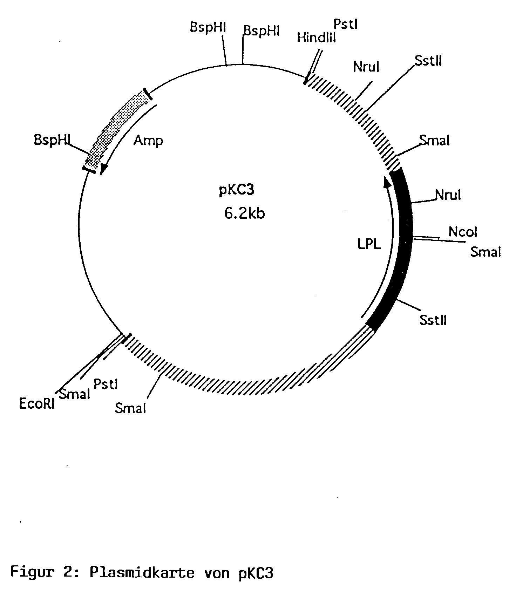

Ein erfindungsgemäßer Vektor ist z.B. das Plasmid pKC3 (siehe Figur 2). Das Plasmid besteht aus dem allgemein bekannten E. coli-Plasmid pUC19 sowie einem HindIII/EcoRI-Restriktionsfragment aus der chromosomalen DNA von A. foetidus RH3046. Das Restriktionsfragment enthält das Strukturgen der LPL mit der natürlichen, für das Signalpeptid codierenden DNA-Sequenz sowie den natürlichen 5' vor dem Gen liegenden Promotor- und 3' hinter dem Gen liegenden Terminationssequenzen. Die vor und hinter dem Strukturgen einschließlich der Signalpeptidsequenz liegenden DNA-Sequenzen sind funktionell und führen zur Expression und Sekretion in filamentösen Pilzen der Gattung Aspergillus, z.B. in Stämmen von A. foetidus, A. niger, A. phoenicis, A. oryzae oder A. sojae.

Dem Fachmann sind hinreichend Methoden bekannt, mit deren Hilfe ein Gen mit funktionellen DNA-Sequenzen, z.B. einem anderen Promotor oder einer Signalpeptidsequenz, in einem Vektor kombiniert werden kann (siehe z.B. Maniatis et al., (1982), Molecular Cloning, A Laboratory Manual, Cold Spring Harbor Laboratory, New York). Die Nukleotidsequenz für das reife LPL-Gen oder eine davon abgeleitete Nukleotidsequenz kann daher auch mit anderen funktionellen Sequenzen als den in pKC3, pKC9 oder pKC12 vorhandenen funktionellen DNA-Sequenzen verbunden werden, die zur Expression und Sekretion der LPL in einem filamentösen Pilz der Gattung Aspergillus oder in T. reesei geeignet sind.

Von besonderer Bedeutung für die Expressionshöhe sind vor allem funktionelle Promotorsequenzen. Hierbei handelt es sich um DNA-Sequenzen von ca. 500 bis 2000 Basenpaaren Länge, die jeweils 5' vor dem Startcodon eines Aspergillus- oder eines T. reesei-Gens liegen.

Solche DNA-Sequenzen können z.B. als Restriktionsfragmente isoliert werden und mit Restriktionsfragmenten des LPL-Gens einschließlich der Signalpeptidsequenz ligiert werden. Dabei können nichtkompatible Restriktionsspaltstellen oder Bereiche ohne geeignete Restriktionsspaltstellen z.B. durch synthetisch hergestellte Oligonukleotide überbrückt bzw. ersetzt werden, so daß die ursprüngliche DNA-Sequenz erhalten bleibt. Auf diese Weise kann die DNA-Sequenz des LPL-Gens einschließlich des Signalpeptids unverändert erhalten bleiben und mit einer ebenfalls unveränderten Promotorsequenz funktionell verbunden werden.

Zur Erhöhung der Expression kann der natürliche Promotor gegen einen anderen ausgetauscht werden. Es sind zahlreiche geeignete Promotoren bekannt. Geeignet ist z.B. der TAKA-Amylase-Promotor aus A. oryzae (siehe EP 0 238 023) zur Expression in A. oryzae- oder A. sojae-Stämmen, der gpdA-Promotor aus A. nidulans (PUNT et al. (1987), Gene 56, S. 117-124) zur Expression in A. nidulans-, A. niger-, A. phoenicis-, A. japonicus-, A. foetidus- oder A. awamori-Stämmen. Geeignet für die Expression in einem T. reesei-Stamm ist z.B. der cbh1-Promotor aus T. reesei (EP-A-0 244 234).

Als Terminator wird die natürliche, hinter dem Strukturgen liegende chromosomale Terminator-Sequenz gemäß SEQ ID NO 1 bevorzugt. Je nach dem zur Expression verwendeten Stamm oder der verwendeten Promotorsequenz kann es von Vorteil sein, zusätzlich die natürliche Leadersequenz oder auch die Terminationssequenz auszutauschen, um eine nochmals verbesserte Expression und Sekretion zu erreichen. Geeignet ist z.B. der trpC-Terminator aus A. nidulans (PUNT et al., a.a.O.) oder der Pektinesterase-Terminator aus A. niger (EP 0 388 593).

<u>Transformierte Aspergillus- oder Trichoderma-Stämme zur großtechnischen Produktion von LPL</u>

Die erfindungsgemäßen Plasmide können bei der Transformation von Aspergillus- oder Trichoderma reesei-Stämmen verwendet werden. Die Plasmide können dabei mehrfach ins Genom der Wirtsstämme integriert werden. Durch die Erhöhung der Anzahl der Genkopien und/oder die zusätzliche Verwendung stärkerer Promotoren kann die LPL-Produktivität bedeutend erhöht werden. Aus einer Vielzahl von Transformanten können solche mit besonders hoher Produktivität ausgewählt werden. Durch die Förderung der LPL-Produktivität treten zugleich Nebenaktivitäten, wie eine unerwünschte Glucoamylase-Aktivität, in den Hintergrund. Erfindungsgemäß transformierte Stämme eignen sich daher besonders gut zur großtechnischen Produktion von LPL-Enzymprodukten.

Besonders geeignet zur Expression und Sekretion der LPL sind Pilzstämme von Arten, deren gute Produktionseigenschaften für Enzyme bekannt sind. Bevorzugt sind insbesondere Stämme der Arten A. niger, A. awamori, A. phoenicis, A. japonicus, A. foetidus, A. oryzae oder A. sojae sowie T. reesei. Geeignete Stämme können aus öffentlich zugänglichen Stammsammlungen, wie z.B. ATCC, DSM, CBS oder NRRL, erhalten werden. Geeignete Wirtsstämme sind z.B. insbesondere A. awamori ATCC 11360, A. foetidus ATCC 10254 und ATCC 11359, A. japonicus 16873, A. oryzae NRRL 695, A. niger ATCC 10864, A. phoenicis CBS 136.52 sowie T. reesei ATCC 26921. Besonders geeignet ist A. sojae DSM 10090. Ebenfalls besonders geeignet ist der Gendonorstamm A. foetidus RH3046 (vgl. Seite 3).

Die Transformation der Pilzstämme kann mit Hilfe bekannter Methoden vorgenommen werden. EP 0 184 438 (US 4 885 249) beschreibt z.B. eine Transformationsmethode für A. niger, bei der als Selektionsmarker das argB-Gen aus A. nidulans verwendet wird. EP 0 238 023 beschreibt im Beispiel 9 eine generell anwendbare Transformationsmethode für A. oryzae-Stämme. Dabei wird das Plasmid p3SR2 mit dem amdS-Gen aus A. nidulans als Selektionsmarker verwendet. EP 0 244 234 beschreibt die Transformation von T. reesei mit diesem Vektor. Die Transformation von A. niger mit p3SR2 wird von Kelly und Hynes (1985), EMBO Journal 4, S. 475-479, beschrieben. Für Stämme der Arten A. niger, A. awamori, A. japonicus, A. phoenicis und A. foetidus kann bevorzugt der Vektor pAN7-1 (PUNT et al. (1987), Gene 56, S. 117-124) verwendet werden. Transformanten können dann anhand der Resistenz gegen Hygromycin B selektiert werden.

Für die Transformation von A. sojae wird ein drittes Selektionsprinzip genutzt, da die verwendeten Stämme dieser Art sowohl Acetamid verwerten wie auch gegen Hygromycin B resistent sind. Durch Selektion auf chlorhaltigem Nährboden werden Mutanten isoliert, deren Nitratreduktase-Gen (niaD) defekt ist, die also nicht mehr mit Nitrat als alleiniger Stickstoffquelle wachsen (Cove, D.J. (1976) Heredity 36, 191-203). Durch Transformation mit dem Plasmid pSTA10 (Unkles, S.E. et al. (1989) Mol. Gen. Genet. 218, 99-104), auf dem sich die intakte Information für das Nitratreduktase-Gen befindet, wird der Defekt ausgeglichen, so daß die Transformanten mit Nitrat als alleiniger Stickstoffquelle wachsen, während die nicht transformierten Zellen im Wachstum zurückbleiben.

Zur Transformation werden zunächst aus vegetativen Zellen oder auch aus Konidiosporen unter Enzymeinwirkung Protoplasten in einem osmotisch stabilisierten Medium erzeugt. Zu diesem werden üblicherweise nach mehreren Waschschritten die zu transformierende Plasmid-DNA in Gegenwart von Polyethylenglykol (PEG) und $CaCl_2$ zugegeben, was zur Aufnahme der DNA in die Zellen führt. Nach der Transformation werden die Protoplasten auf osmotisch stabilisiertem Medium regeneriert, wobei eine Selektion auf solche Zellen erfolgt, die ein Markergen aufgenommen haben.

Die Transformation der Pilzstämme erfolgt bevorzugt nach dem Co-Transformationsverfahren, bei dem ein Plasmid mit einem Selektionsmarker für Aspergillus- oder T. reesei-Stämme und ein erfindungsgemäßer Vektor - eine Phagen-DNA oder eine Cosmid-DNA oder bevorzugt ein Plasmid mit dem LPL-Gen - gleichzeitig zu den zu transformierenden Zellen zugegeben werden.

A. sojae DSM 10090 kann beispielsweise mit den Plasmiden pSTA10 und pKC9 co-transformiert werden. Es können dann nitratverwertende Transformanten selektiert werden.

Unter den insgesamt erhaltenen Transformanten müssen dann solche mit erhöhter oder besonders hoher LPL-Produktivität ausgewählt werden. Diese Auswahl kann z.B. aufgrund der LPL-Produktivität der Transformanten in Schüttelkolben getroffen werden.

## Verfahren zur Herstellung von LPL mit einem erfindungsgemäß transformierten Wirtsorganismus

Der transformierte Wirtsstamm kann in einem geeigneten Medium in Submerskultur inkubiert werden. Geeignete Medien sind solche, in denen filamentöse Pilze ein gutes Wachstum zeigen, insbesondere solche, in denen zugleich eine gute Bildung der LPL erfolgt. Gut geeignet sind Medien, von denen bekannt ist, daß der jeweilige Aspergillus- oder T. reesei-Wirtsstamm ein gutes Wachstum zeigt und zugleich LPL bildet. Besonders günstig auch im Hinblick auf kostengünstige Medien ist die Verwendung billiger Naturprodukte als Nährbodenbestandteile, wie z.B. Maisquellpulver oder Maisquellwasser, Roggenkleie, Weizenkleie, Kartoffeldextrin, Maltodextrin, Kartoffelprotein, Melasse etc.. Günstig ist ebenfalls die Verwendung von Ammoniumsalzen als N-Quellen, z.B. Ammoniumsulfat. Der Fachmann kann sich an für die Fermentation von Aspergillus- oder Trichoderma reesei-Stämmen bekannten Medien orientieren und durch Versuche besonders geeignete Medien herausfinden. Ein geeignetes Medium kann z.B. 5 % Maltodextrin und 3 % Maisquellpulver in Leitungswasser enthalten.

Die Herstellung der LPL kann durch Submersfermentation erfolgen. Die Beimpfung kann dabei üblicherweise über eine Impfkaskade von einer Kulturröhrchen- oder einer Petrischalenkultur über Schüttelkolben, ggf. einen oder mehrere Vorfermenter in einen Hauptfermenter erfolgen. Eine übliche Fermentationsdauer beträgt ca. 30 bis 120 Stunden bei ca. 28°C unter aeroben Bedingungen. Nach Abbruch der Fermentation wird das Zellmaterial, z.B. durch Filtration abgetrennt und der LPL-haltige Kultursaft geerntet und gereinigt. Der Kultursaft kann zu flüssigen oder trockenen LPL-Produkten, z.B. durch Ultrafiltration, Sprühtrocknung oder Sprühgranulation, weiterverarbeitet werden. Auf diese Weise können LPL-Enzymprodukte hergestellt werden, die zur Hydrolyse von Lysophosphatiden geeignet sind.

## Anwendung des erfindungsgemäßen LPL-Enzymproduktes

Die Hydrolyse-Reaktion wird am besten im Temperaturbereich von 20 bis 65°C, vorzugsweise von 35 bis 55°C, in 5 bis 24 Stunden unter Rühren durchgeführt. Zweckmäßig werden die Ausgangssirupe in einer Aufschlämmung oder Lösung mit einem Festgehalt von 5 bis 35 Gew.-% eingesetzt. Der pH-Wert liegt im allgemeinen im Bereich von 4 bis 5, vorzugsweise bei 4,5.

Vorzugsweise wird das Verfahren einstufig durchgeführt, indem man die LPL einwirken läßt. Zweckmäßig sind pro kg Trockensubstanz des Sirups 250 bis 800 Einheiten an LPL. Dabei können unter Verwendung der rekombinanten LPL wesentlich höhere Flußraten erzielt werden als dies etwa mit der in EP 0 219 269 beschriebenen konventionellen LPL möglich ist.

Durch Pasteurisieren des Sirups wird üblicherweise das Enzym LPL inaktiviert.

Die folgenden Beispiele erläutern die Erfindung näher.

**Beispiele**

(Prozentangaben in den Beispielen bedeuten Gew.-% soweit nicht anders angegeben.)

Beispiel 1

Reinigung der LPL aus A. foetidus

1500 ml Kulturretentat von A. foetidus RH3046 wurden, um die elektrische Leitfähigkeit herabzusetzen, über eine Sephadex G25-Säule (Pharmacia) entsalzt und auf Puffer A (20 mM Tris-HCl, pH 7,0 + 5 mM Ca-Acetat) umgepuffert. Die aufgefangene Enzymlösung hatte eine Leitfähigkeit von 3,5 mS/cm.

In einem weiteren Schritt wurde eine Ionenaustauschchromatographie an DEAE-Fractogel (Merck) vorgenommen. Dazu wurden 2440 ml der umgepufferten entsalzten Enzymlösung in mehreren (9) Ansätzen auf eine DEAE-Fractogel-Säule (Höhe 235 mm, Durchmesser 50 mm) aufgetragen. Die Säule wurde mit Puffer A gespült. Die Elution erfolgte in einem kontinuierlichen Gradienten von Puffer A zu Puffer B (Puffer A + 1 M NaCl). Es wurde bei einer Elutionsgeschwindigkeit von 12 ml/min eluiert, und Fraktionen zu je 18 ml wurden aufgefangen.

Die Fraktionen wurden auf Anwesenheit der LPL getestet. Dies geschah durch Messung der LPL-Aktivität. Eine LPL-Einheit ist danach definiert als die Enzymmenge, die in einer 0,010 molaren wäßrigen Lösung von Lysolecithin bei pH 4,5 und 55°C eine Hydrolysegeschwindigkeit von 1 Mikromol pro Minute bewirkt.

Die Messung der LPL-Aktivität wurde wie folgt ausgeführt: 0,1 ml Enzymlösung wurden zu 0,5 ml bei 55°C vortemperierter Substratlösung (0,01 M Lysolecithin, Sigma Best.-Nr. L4129, in 0,01 M Na-Acetat-Puffer, pH 4,5) gegeben. Nach 1 min und nach 10 min Reaktionszeit bei 55°C wurden jeweils 0,1 ml dieses Ansatzes zu 1 ml auf 37°C vortemperiertes (5 min) Reaktionsgemisch A (Test der Fa. Boehringer Mannheim, "Freie Fettsäuren, Halbmikrotest", Best.-Nr. 1383175; Reaktionsgemisch A enthält 11 ml K-Phosphat-Puffer, pH 7,8, Tribromhydroxy-Benzoesäure, Mg-Chlorid und Stabilisatoren, sowie eine Tablette, die ATP, CoA, Acyl-CoA-Synthetase, Peroxidase, Ascorbat-Oxidase, 4-Aminoantipyrin und Stabilisatoren enthält) gegeben und bei 37°C 5 min inkubiert. Anschließend wurden 0,1 ml des Gemisches aus 0,55 ml N-Ethylmaleinimidlösung (im Testkit enthalten) und 0,55 ml Reaktionsgemisch B (im Boehringer-Testkit enthalten, 0,6 ml ACOD-Verdünnungslösung, stabilisiert, sowie eine Tablette Acyl-CoA-Oxidase) zu obigem Ansatz zugegeben und 5 min bei 37°C inkubiert. Der Ansatz wird im Wasserbad auf Raumtemperatur abgekühlt und die Extinktion bei 546 nm im Photometer bestimmt (1-ml-Küvetten). Die Enzymlösung wurde mit destilliertem Wasser so verdünnt, daß die Extinktionswerte im Bereich von 0,5 erhalten wurden. Der Gehalt an LPL wurde nach folgender Formel errechnet.

Berechnung:

$$\frac{E \text{ (nach 10 min)} - E \text{ (nach 1 min)} \cdot 1,2 \text{ ml} \cdot 0,6 \text{ ml}}{9 \text{ min} \cdot 16,772 \cdot 1 \text{ cm} \cdot 0,1 \text{ ml} \cdot 0,1 \text{ ml} \cdot \text{Enzymkonz. g/ml}}$$

Einheit: U/g LPL
16,772 = OD von 1 mM Palmitinsäure, gemessen nach oben beschriebenem Test
Analysenvolumen = 1,2 ml
Volumen Enzymlösung = 0,1 ml
Volumen der freien Fettsäure = 0,1 ml
Enzymkonzentration = Verdünnung der Enzymlösung $\cdot$ Enzymeinwaage (g/ml)
Reaktionszeit $t_{min}$ = 9 (10-1) min
Schichtdicke der Meßküvette = 1 cm
Volumen Puffer + Substrat + Enzym = 0,6 ml

Die Elution der LPL setzte bei ca. 0,1 M NaCl ein. Die LPL-haltigen Fraktionen aus mehreren Läufen wurden vereinigt, gegen $H_2O_{dest.}$ dialysiert und anschließend lyophilisiert. Das Lyophilisat wurde in 168 ml Puffer A aufgenommen. Im nächsten Schritt wurde je 1 ml der Proben in 168 Ansätzen auf eine Mono Q (Pharmacia, 10 ml)-Anionenaustausch-Chromatographie-Säule aufgetragen und wiederum in einem kontinuierlichen Gradienten von Puffer A zu Puffer B eluiert. Die Hauptmenge an LPL eluierte dabei zwischen 180 und 210 mmol NaCl. Das Eluat, 4035 ml, wurde gegen $H_2O_{dest.}$ dialysiert und anschließend lyophilisiert.

Das Lyophilisat wurde in 100 ml Puffer A aufgenommen und in 5 Läufen à 20 ml über eine Sephacryl S-200 HR (Pharmacia)-Säule (770 mm $\cdot$ 50 mm) durch Gelchromatographie weiter gereinigt.

Die LPL-haltigen Fraktionen (300 ml) wurden vereinigt und mit 300 ml 3,4 M Ammoniumsulfatlösung versetzt und in 15 Läufen mit je 40 ml über eine Phenyl-Sepharose (Pharmacia, FF, 83 mm $\cdot$ 10 mm) getrennt, mit 1,7 M Ammoniumsulfatlösung in Puffer A (Puffer C) nachgewaschen und mit einem kontinuierlichen Gradienten von Puffer C und Puffer A eluiert. Die LPL-haltigen Fraktionen (85 ml) wurden vereint, die spezifische Aktivität lag bei ca. 72 LPL Einheiten/mg Protein.

Endreinigung der LPL mittels HPLC

Die Endreinigung erfolgte durch Umkehrphasenchromatographie (HPLC) an Nucleosil 500-5 C3 PPN (Standard-Prep Trennsäule, Macherey und Nagel). Je 1350 μl der LPL-haltigen Fraktionen wurden aufgetragen. Die Elution erfolgte mit einem Ammoniumformiat-Puffersystem (Puffer A: 10 mM Ammoniumformiat, pH 7,5 (95 %), Acetonitril (5 %) und Puffer B: 100 mM Ammoniumformiat, pH 7,5 (5 %), Acetonitril (95 %), 9 auf 10, bis 80 % Puffer B. Die LPL eluierte bei ca. 47 % Puffer B.

Das gereinigte Protein zeigt in der SDS-Gelelektrophorese eine einheitliche Bande mit einem Molekulargewicht von ca. 37.000 Dalton. Der isoelektrische Punkt liegt bei ca. pH 4,2. Das auf diese Weise gereinigte Protein wurde zur Sequenzierung verwendet.

Beispiel 2

Partielle Aminosäuresequenzierung

Ausgehend von dem gereinigten Enzym, wurden nach der Spaltung mit Endoproteinase Asp-N die Peptide durch Umkehrphasen-HPLC-Chromatographie getrennt. Die N-terminale Sequenz des nativen Proteins wie die Sequenz der präparierten Peptide wurden unter Verwendung eines Gasphasensequenators (Applied Biosystems Model 470A) bestimmt.

Die N-terminale Aminosäuresequenz lautet:

Ser-Val-Ser-Thr-Ser-Thr-Leu-Asp-Glu-Leu-Gln-Leu-Phe-Ala-Gln-Trp-Ser-Ala-Ala-Ala-Tyr-Cys-Ser-Asn-Asn-Ile-Asp-Ser.

Das nach Endoproteinase Asp-N (Boehringer Mannheim)-Spaltung erhaltene Peptid Asp-N3 mit folgender Aminosäuresequenz wurde zur Ableitung des Oligonukleotids KC15 benutzt:

Asp-N3:    Glu-His-Ile-Thr-Ser-Gln-Gly-Ser-Gly-Ala-Asn-Phe-Arg
KC15:      3'-CTC GTG TAG TGG AGG GTC CCG AGG CGG-5'

Beispiel 3

Ableitung und Synthese von DNA-Sonden

Die Sequenz des KC13-Oligonukleotids wurde aus der N-terminalen Aminosäuresequenz der Lysophospholipase abgeleitet und am 5'-Ende im Sinne des Oligonukleotids TCCAAGCTT geändert, um eine HindIII-Schnittstelle zu erhalten.

Das Oligonukleotid KC13 stellt eine sogenannte "mixed-probe" dar. Seine Sequenz lautet:

```
N-Terminus:     Ser-Thr-Leu-Asp-Glu-Leu-Gln-Leu-Phe-Ala-Gln-
KC13:        5'-TCC AAG CTT GAC GAA CTI CAA CTI TTC GCI CA-3'
                             T   G T     G T     T
            HindIII
```

Zur Identifizierung des PCR-Produkts bzw. für Northern-Analysen und zur Identifizierung von Kolonien wurde das aus dem Peptid-Fragment Asp-N3 abgeleitete Oligonukleotid KC15 verwendet.

KC15:    3'-CTC GTG TAG TGG AGG GTC CCG AGG CGG-5'

Die Synthese der Nukleotidsequenz erfolgte nach dem von Beaucage, S.L. und Caruthers, M.H., (1981), Tetrahedron Letters 22, S. 1859-1862, entwickelten Phosphoramiditverfahren und wurde mittels eines DNA-Synthesegeräts (Applied Biosystems 380 A DNA Synthesizer) durchgeführt.

Beispiel 4

Präparation von RNA aus den induzierten Zellen

Zur Isolierung von RNA aus Aspergillus foetidus wurde eine modifizierte Methode nach Vierula, P.J. und Kapoor, M., (1989), J. Biol. Chem. 264, S. 1108-1114, angewandt.

Beispiel 5

Synthese von cDNA und Isolierung eines LPL-spezifischen cDNA-Klons

Die cDNA-Synthese wurde nach der Methode von Russo et al., (1991), EMBO J. 10, S. 563-571, durchgeführt. Das Prinzip dieses Verfahrens beruht darauf, zunächst durch einen Oligo(dT)-Primer (EA13) sämtliche polyadenylierte RNA-Moleküle in einzelsträngige cDNA umzuschreiben. An diese cDNA-Synthese schließt sich eine PCR-Reaktion an. Sie wird gestartet durch einen sequenzspezifischen Primer (KC13). Die Rückreaktion erfolgt durch eine Teilsequenz des Oligo(dT)-Primers (EA14). In diese Sequenz sind Erkennungsschnittstellen für Restriktionsenzyme bereits eingebaut.

Die Sequenz des EA13- bzw. EA14-Oligonukleotids lautet:

```
EA13:   5'-GAC TCG AGT CGA CAT CGA (T)₂₁ A/C/G-3'


EA14:   5'-GAC TCG AGT CGA CAT CGA TT-3'
              XhoI    SalI
```

Bedingungen für die Synthese der einzelsträngigen cDNA

Für die Synthese des ersten Stranges wurden in einem Reaktionsvolumen von 50 $\mu$l 10 $\mu$g Gesamt-RNA, 50 mM Tris-HCl, pH 8,3, 5 mM MgCl$_2$, 75 mM KCl, 5 mM DTT, 0,4 mM dNTP (je 0,4 mM dATP, dCTP, dGTP, dTTP), 30 pmol EA13 und 200 U M-MLV reverse Transkriptase (Gibco-BRL GmbH, 7514 Eggenstein, BRD) eingesetzt. Es wurde für 45 min bei 42°C inkubiert.

Versuchsbedingungen für die PCR-Reaktion

Für die Polymerase-Ketten-Reaktion wurden in einem Reaktionsvolumen von 100 $\mu$l 20 mM Tris-HCl, pH 8,4, 50 mM KCl, 1,5 mM MgCl$_2$, 0,2 mM dNTP (je 0,2 mM dATP, dCTP, dGTP, dTTP), 70 pmol EA14, 77 pmol KC13, 2 $\mu$l vom ersten Strang cDNA-Synthese-Reaktionsansatz und 5 U Taq-DNA-Polymerase (Gibco-BRL) eingesetzt. Der Ansatz wurde für 20 Zyklen (94°C, 40 sek; 50°C, 2 min; 72°C, 3 min) durchgeführt.

Nach der gelelektrophoretischen Auftrennung der PCR-Produkte konnte ein Fragment von ca. 0,9 kb identifiziert werden, das mit dem Oligonukleotid KC15 hybridisierte. Die PCR-Produkte wurden mit Ethanol gefällt, mit HindIII/SalI gespalten und in das Plasmid pUC18 insertiert.

Es wurden 195 weiße Kolonien erhalten. Davon wurden 48 Kolonien ausgewählt, und eine Schnellplasmidpräparation wurde durchgeführt. Nach der Hybridisierung mit dem Oligo-KC15 zeigten neun Kolonien mit der Bezeichnung pKC1/a Nr. 5, 16, 17, 22, 27, 30, 36, 43 und 46 eindeutige Signale. Die Plasmide der Klone Nr. 5 und 36 wurden präpariert und sequenziert.

Die von der Nukleotidsequenz abgeleitete Aminosäuresequenz stimmt mit der durch Edman-Abbau bestimmten Sequenz überein.

Das Plasmid pKC1/36 enthielt allerdings nur die Nukleotid- und Aminosäuresequenz der Lysophospholipase-cDNA ab Codon 29. Um Informationen über die Signalsequenz und darüber hinaus die Intron/Exon-Verteilung in der genomischen DNA genau festlegen zu können, wurde die Klonierung des gesamten cDNA-Gens mittels der PCR-Methode durchgeführt. Die Isolierung der RNA, die Synthese der einzelsträngigen cDNA und die Bedingungen für die Polymerase-Ketten-Reaktion wurden wie vorher bei der Klonierung des Plasmids pKC1/36 beibehalten. Geändert wurde jedoch die Verwendung des Oligonukleotids KC24 anstelle des Oligonukleotids KC13 als Primer bei der Polymerase-Ketten-Reaktion. Das Oligonukleotid KC24 wurde aus dem genomischen Lysophospholipase-Gen abgeleitet und liegt ab Nukleotid -50 vor dem Startcodon ATG. Seine Sequenz (KC24) lautet: 5'-GTT GGT CGC AAG GTT TTG-3'.

Das PCR-Produkt wurde mit AccI gespalten und in das mit dem gleichen Enzym gespaltene Plasmid pUC18 insertiert. Als Gensonde zur Identifizierung von Kolonien wurde das HindIII/SalI-Fragment des Plasmids pKC1/36 verwendet. Das neue rekombinante Plasmid mit der Bezeichnung pKC/ATG wurde sequenziert. Die Sequenz der Insertion ist in der SEQ ID NO 1 dargestellt.

Beispiel 6

Isolierung des chromosomalen Lysophospholipase-Gens

Die Isolierung des chromosomalen Lysophospholipase-Gens erfolgte durch direkte Klonierung der mit Restriktionsenzymen hydrolysierten chromosomalen DNA in den Vektor pUC18. Zuerst wurde die chromosomale DNA aus A. foetidus RH3046 isoliert, mit Restriktionsenzymen fragmentiert und in einer Southern-Analyse charakterisiert. Als Hybridisierungssonde wurde das HindIII/SalI-Fragment aus dem Plasmid pKC1/36 verwendet. Aufgrund des Ergebnisses aus der Southern-Hybridisierung wurde die chromosomale DNA von A. foetidus RH3046 mit BamHI vollständig hydrolysiert und anschließend in einem Saccharose-Gradienten nach Größe fraktioniert. Fraktionen, die DNA-Fragmente der Größe von ca. 9 bis 10 kb enthielten, wurden gesammelt, mit Ethanol gefällt und in das mit BamHI gespaltene Plasmid pUC18 insertiert. Es wurden 1904 rekombinante Klone erhalten. Davon konnten 24 positive Transformanten mittels Koloniehybridisierung identifiziert werden. Die Plasmid-DNA von Transformant Nr. 1 und Nr. 3 wurde präpariert, kartiert, erneut hybridisiert, und anschließend wurden die den N- und C-terminalen Bereichen des Proteins entsprechenden Bereiche des Gens sequenziert. Das Ergebnis bestätigte, daß beide Klone das chromosomale Lysophospholipase-Gen enthalten. Dieses neue Plasmid trägt die Bezeichnung pKC6.

Da das Plasmid pKC6 einen sehr großen 5'- bzw. 3'-nichttranslatierten Bereich enthielt, wurde aus dem Plasmid pKC6 das PstI-Fragment isoliert und in das mit PstI gespaltene Plasmid pUC18 insertiert. Das neue Plasmid erhielt die Bezeichnung pKC3. Die gesamte Insertionssequenz des Plasmids pKC3 ist in SEQ ID NO 1 dargestellt. Durch den Vergleich der genomischen DNA mit der Lysophospholipase-cDNA konnten zusätzliche 1608 Nukleotide stromaufwärts vom ATG bei der genomischen DNA festgestellt werden, so daß sowohl die cDNA-Sequenz wie die genomische Sequenz des Lysophospholipase-Strukturgens bestimmt wurde. Das genomische Gen enthält drei Introns. Der 3'-nichtcodierende Bereich umfaßt 997 Nukleotide. Die durch Computer-Analyse ermittelte Signalsequenz enthält 21 Aminosäuren. Vermutlich wird das Lysophospholipase-Gen als Präproprotein synthetisiert, wobei das Pro-Peptid sechs Aminosäuren umfaßt.

Beispiel 7

Konstruktion des Expressionsvektors pKC9

Der Vektor pKC9 enthält das genomische Lysophospholipase-Gen und den Aspergillus oryzae-$\alpha$-Amylase-Promotor aus dem Plasmid pK54. Um den $\alpha$-Amylase-Promotor vor das Lysophospholipase-Gen zu setzen, mußte die Initiationssequenz TA TAC CGC AAG ATG TTC gegen die Sequenz AC CTG CTA ACC ATG TTC, welche die Erkennungssequenz für BspMI enthält, ausgetauscht werden. Dazu wurde die Polymerase-Ketten-Reaktion (PCR) benutzt. Es wurden zwei Oligonukleotid-Primer mit den folgenden Sequenzen verwendet:

```
KC29:
5'-GGA ATT CAC CTG CTA ACC ATG TTC TCT GGA CGG TTT GGA GTG-3'
                 BspMI


SupI:
5'-CGC CAG GGT TTT CCC AGT CAC GAC-3'
```

Für die Polymerase-Ketten-Reaktion wurden in einem Reaktionsvolumen von 100 µl 20 mM Tris-HCl, pH 8,4, 50 mM KCl, 1,5 mM MgCl , 0,2 mM dNTP (je 0,2 mM dATP, dCTP, dGTP, dTTP), je 50 pmol KC29 und SupI, 1 ng pKC3 als Matrize und 2,5 U Taq-Polymerase (Perkin-Elmer) vermischt. Der Ansatz wurde für 20 Zyklen (94°C, 40 sek; 40°C, 2 min; 72°C, 2 min) durchgeführt. Nach Beendigung der Reaktion wurde das amplifizierte Fragment gereinigt, mit BspMI und HindIII hydrolysiert und in das mit NcoI/HindIII gespaltene Plasmid pK54 insertiert. Die Konstruktion des Plasmids pKC9 wurde durch eine Restriktionsanalyse und die anschließende Sequenzierung bestätigt.

<u>Konstruktion von Expressionsvektor pKC12</u>

In das Plasmid pKC12 wurde das Lysophospholipase-Gen unter der Kontrolle des Aspergillus niger Xylanase-Promotors eingesetzt. Die Konstruktion des Plasmids pKC12 wurde in gleicher Weise wie die des Plasmids pKC9 durchgeführt. Nach Beendigung der Polymerase-Ketten-Reaktion (PCR) wurde das amplifizierte Fragment, das das Lysophospholipase-Gen enthält, mit BspMI und HindIII hydrolisiert und in das mit NcoI/HindIII geschnittene Plasmid pxylP, das den Aspergillus niger Xylanase-Promotor enthält, insertiert.

Die Konstruktion des Plasmids pKC12 wurde durch eine Restriktionsanalyse und anschließender Sequenzierung bestätigt.

<u>Beispiel 8</u>

<u>Transformationsmethode für Aspergillus- und Trichoderma reesei-Stämme</u>

Von einer ca. zwei Wochen alten Petrischalenkultur des zu transformierenden Pilzstammes wurde eine Sporensuspension in ca. 10 ml 0,85 % NaCl durch Abschwemmen unter Zuhilfenahme eines Spatels hergestellt. Es wurden je vier 1-l-Schüttelkolben mit 100 ml Czapek-Dox-Minimalmedium (Oxoid) mit 0,1 % Hefeextrakt mit je 1 ml Sporensuspension beimpft und ca. 16 Stunden bei 28°C auf einem Rundschüttler bei 120 Umdrehungen pro Minute inkubiert. Das Mycel aus je vier Schüttelkolben wurde über einem Papierfilter geerntet und mit ca. 50 ml MP-Puffer (1,2 mM $MgSO_4$ in 10 mM Phosphatpuffer, pH 5,8) gespült. Nach dem Ablaufen des Puffers wurde das feuchte Mycel gewogen. In der Regel wurden ca. 3 bis 5 g Feuchtmycel erhalten.

Pro g Feuchtmycel wurden 5 ml MP-Puffer, 120 $\mu$l Novozym-Lösung (1 g Novozym$^®$ 234 (Novo Nordisk) in 6 ml MP-Puffer) und 25 $\mu$l $\beta$-Glucuronidase (Sigma) zugegeben. Die Mycel-Suspension wurde 5 min in Eiswasser gestellt. Anschließend wurden 60 $\mu$l Rinderserumalbumin-Lösung (0,2 g Rinderserumalbumin in 4 ml MP-Puffer, sterilfiltriert) zugegeben, und der Ansatz wurde unter leichtem Schütteln bei 30°C inkubiert. Die Bildung von Protoplasten wurde visuell im Mikroskop verfolgt. Als keine wesentliche Zunahme der Protoplastenbildung mehr festgestellt wurde, wurde der Ansatz zur Ernte der Protoplasten abgebrochen. Dies war in der Regel nach etwa 3 bis 5 Stunden der Fall.

Die Protoplastensuspension wurde zur Abtrennung noch vorhandener grober Mycelbestandteile über ein mit MP-Puffer getränktes Glaswollefilter gegeben und in Zentrifugenröhrchen überführt. Die obere Hälfte der Röhrchen wurde mit 600 mM Sorbit, 100 mM Tris-HCl, pH 7,0, überschichtet. Die Röhrchen wurden 10 min bei 2500 g zentrifugiert. Die Protoplasten wurden aus der Zwischenschicht abgenommen und in 1 M Sorbit, 10 mM Tris-HCl, pH 7,5, aufgenommen. Anschließend wurden die Protoplasten zweimal mit STC-Puffer (1 M Sorbit, 10 mM Tris-HCl, pH 7,5, 10 mM $CaCl_2$) durch Zentrifugation bei 1500 g gewaschen und zuletzt in 1 ml STC-Puffer aufgenommen.

Zur Transformation von A. oryzae oder A. foetidus wurden 300 $\mu$l Protoplastensuspension, ca. 10 $\mu$g p3SR2 als Selektionsplasmid und 10 $\mu$g des jeweiligen Plasmids zur Expression der LPL in 25 $\mu$l 10 mM Tris-HCl, pH 8,0, zusammengegeben und 10 min bei 0°C inkubiert. Anschließend wurden nochmals 25 $\mu$l der gleichen DNA-Menge und 400 $\mu$l PEG-Lösung (60 % Polyethylenglykol 6000 (Fluka) in 10 mM Tris-HCl, pH 7,5, 50 mM $CaCl_2$) zusammengegeben, sehr vorsichtig vermischt und 5 min bei Raumtemperatur inkubiert. Es wurden nochmals 600 $\mu$l PEG-Lösung zugegeben, vermischt, und der Ansatz wurde für weitere 20 min bei Raumtemperatur inkubiert. Zu dem Ansatz wurde ca. 9 ml Acetamid-Weichagar (Minimalmedium mit 10 mM Acetamid als einziger N-Quelle, 1 M Saccharose, 0,6 Gew.-% Agar) bei 45°C zugemischt und auf vier Petrischalen mit dem gleichen Medium, jedoch mit 1,5 Gew.-% Agar (Oxoid) und zusätzlich 15 mM CsCl, verteilt. Die Platten wurden bei 28°C inkubiert. Nach 6 bis 10 Tagen wurden schnell wachsende Kolonien (Transformanten) auf Acetamid-Medium ohne Saccharose isoliert, zweimal über Einzelsporkolonien gereinigt und zuletzt auf Vollmedium, z.B. Kartoffel-Dextrose-Agar, übertragen.

Die Transformation von Stämmen der Arten A. niger, A. awamori, A. foetidus, A. japonicus oder A. phoenicis kann ebenfalls mit dem Plasmid p3SR2 erfolgen. Bevorzugt wurde die Transformation jedoch mit dem Plasmid pAN7-1 ausgeführt. Die Protoplastenpräparation und die Zugabe von Plasmid-DNA erfolgt in analoger Weise, wie oben für das Plasmid p3SR2 beschrieben. Statt der Zugabe von Acetamid-Weichagar wird jedoch der gesamte Transformationsansatz zu 100 ml Czapek-Dox-Minimalmedium (Oxoid) mit 100 $\mu$g Hygromycin B/ml, 1,5 Gew.-% Agar (Oxoid) und 1 M Saccharose, abgekühlt auf ca. 45°C, gegeben und vorsichtig vermengt. Der Ansatz wird dann in Portionen zu je 10 ml in Petrischalen gegeben, in denen jeweils 10 ml Czapek-Dox-Minimalmedium (Oxoid) mit 1,5 Gew.-% Agar (Oxoid), jedoch ohne Hygromycin und ohne Saccharose als feste Unterschicht, vorgelegt war. Nach dem Erstarren der oberen Agarschicht werden die Petrischalen bei 37°C inkubiert. Gegen Hygromycin B resistente Transformanten können nach ca. 3 bis 10 Tagen abgeimpft werden und zur Überprüfung der Resistenz auf Czapek-Dox-Minimalmedium (Oxoid) mit 50 $\mu$g Hygromycin B/ml und 1,5 Gew.-% Agar (Oxoid) übertragen werden.

Für die Transformation von A. sojae wird ein drittes Selektionsprinzip genutzt, da die verwendeten Stämme dieser Art sowohl Acetamid verwerten wie auch gegen Hygromycin B resistent sind. Durch Selektion auf chlorathaltigem Nährboden werden Mutatanten isoliert, deren Nitratreduktase-Gen (niaD) defekt ist, die also nicht mehr mit Nitrat als alleiniger Stickstoffquelle wachsen (Cove, D.J. (1976) Heredity 36, 191-203). Durch Transformation mit dem Plasmid pSTA10 (Unkles, S.E. et al. (1989) Mol. Gen. Genet. 218, 99-104), auf dem sich die intakte Information für das Nitratreduktase-Gen befindet, wird der Defekt ausgeglichen, so daß die Transformanten mit Nitrat als alleiniger Stickstoffquelle wachsen, während die nicht transformierten Zellen im Wachstum zurückbleiben.

Beispiel 9

Herstellung von LPL sezernierenden Transformanten

Homologe Transformation von pKC12 in A. foetidus RH3046

Durch Co-Transformation von A. foetidus RH3046 mit den beiden Plasmiden pAN7-1 (siehe Beispiel 8) und pKC12 in der oben beschriebenen Weise wurden gegen Hygromycin B resistente Transformanten von den Agarschalen mit Regenerationsmedium isoliert. Nach Überprüfung auf Reinheit durch Ausstriche wurden die Transformanten in Schüttelversuchen auf die Produktion von LPL getestet.

250 ml Erlenmeyer-Kolben, gefüllt mit 40 ml Nährlösung, bestehend aus

| Maltodextrin | 3,75 % |
|---|---|
| Maisquellpulver | 3 % |
| $K_2HPO_4$ | 2 % |
| $KH_2PO_4$ | 1,4 % |
| Leitungswasser, 30 min bei 121°C sterilisiert | |

werden mit Sporensuspensionen der zu testenden Transformanten beimpft und bei 28°C, 200 U/min und einer Auslenkung von 25 mm 120 Stunden geschüttelt. Die Zellmasse wurde abfiltriert, im Filtrat der Kulturen wurde die LPL-Aktivität bestimmt. Die Transformante A. foetidus RH3046 pAN7-1 pKC12 Nr. 40 hatte beispielsweise eine um 100 % erhöhte LPL-Aktivität im Kulturfiltrat verglichen mit dem Wirtsstamm A. foetidus RH3046.

Heterologe Transformation von pKC9 in A. sojae RH 3782

Der Stamm A. sojae RH 3782 niaD22, eine nach Cove (1976) hergestellte Mutante von A. sojae RH 3782, wird in der nachfolgenden Nährlösung aus

| Glucose (Merck) | 2 % |
|---|---|
| Malzextrakt (Oxoid) | 0,5 % |
| Bacto-Pepton (Difco) | 0,025 % |
| deionisiertes Wasser | |
| pH-Wert auf 5,0 einstellen; Sterilisation: 30 min bei 121°C | |

angezogen. Aus dem Mycel werden nach der in Beispiel 8 beschriebenen Methodik Protoplasten gewonnen und diese mit pSTA10 als Selektionsplasmid und jeweils einem der Plasmide pKC3, pKC9 oder pKC12 co-transformiert. Zur Regeneration der Protoplasten wird der Transformationsansatz mit 9 ml Weichagar (osmotisch stabilisierend), bestehend aus

| 0,1 M Na-Phosphat-Puffer pH 6,0 | 15 ml |
|---|---|
| 1 M Saccharose (Merck) | 10,28 g |
| Millipore-Wasser auf | 29,1 ml |
| Agar (Oxoid) | 0,18 g (= 0,6 %) |
| 30 min Sterilisation bei 121°C, anschließend sterile Zugabe von: | |
| Salzlösung (7.14.2) | 0,6 ml |
| 1 M NaNO$_3$-Lösung | 0,3 ml |

gemischt und auf vier Agarplatten der gleichen Zusammensetzung, jedoch mit 1 % Agar hergestellt, verteilt. Nach etwa 6-10 Tagen Inkubation bei 37°C werden die Transformanten von den Agarplatten isoliert, auf Nitrat-Saccharose-Agar durch Ausstrich gereinigt. Es wurde eine Vielzahl Transformanten durch Selektion und anschließender Reinigung auf einem Nährboden mit Nitrat als einziger N-Quelle erhalten und in Schüttelkolben unter Verwendung der folgenden Nährlösung

| Maltodextrin | 3,75 % |
|---|---|
| Maisquellpulver | 3,0 % |
| KH$_2$PO$_4$ | 1,0 % |
| K$_2$HPO$_4$ | 0,7 % |
| Triton X-100 | 1,0 % |
| in Leitungswasser, 30 min bei 121°C sterilisiert, | |

auf Produktion von LPL geprüft. Neben Transformanten, die keine oder nur geringe Mengen von LPL - so wie der nichttransformierte Wirt A. sojae RH 3782 bzw. A. sojae RH 3782 niaD22 - produzieren, werden auch solche Transformanten gefunden, die deutlich erhöhte LPL-Aktivität im Kulturfiltrat aufweisen. Diese als Co-Transformanten bezeichneten Stämme eignen sich zur Herstellung des Enzyms.

| Stamm bzw. Transformante | relative LPL-Aktivität |
|---|---|
| A. sojae RH 3782 niaD22 | 100 |
| A. sojae RH 3782 niaD22 pSTA10 pKC3 | 230 |
| A. sojae RH 3782 niaD22 pSTA10 pKC9 | 320 |
| A. sojae RH 3782 niaD22 pSTA10 pKC12 | 220 |

**Patentansprüche**

1. Aus Aspergillus isolierbare rekombinante Desoxyribonukleinsäure (DNA), dadurch **gekennzeichnet,** daß sie für eine Lysophospholipase (LPL) codiert und die in SEQ ID NO 1 für die reife LPL angegebene Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist.

2. Rekombinante Desoxyribonukleinsäure nach Anspruch 1, dadurch **gekennzeichnet,** daß sie aus Aspergillus foetidus isolierbar ist.

3. Vektor, enthaltend

   a) DNA-Sequenzen zur Replikation des Vektors in E. coli,
   b) DNA-Sequenzen zur Expression und Sekretion eines Polypeptids in einem Aspergillus-Stamm oder in einem Trichoderma reesei-Stamm, die für einen Promotor, eine Signalpeptidsequenz und gegebenenfalls für einen Terminator codieren,
   c) eine für ein Polypeptid codierende DNA-Sequenz, die funktionell mit den DNA-Sequenzen nach b) verbunden ist,

   dadurch **gekennzeichnet,** daß die DNA-Sequenz nach c) eine Nukleotidsequenz entsprechend der in für die reife LPL in SEQ ID NO 1 angegebenen Nukleotidsequenz oder eine davon abgeleitete Nukleotidsequenz, die unter stringenten Bedingungen mit der in SEQ ID NO 1 für die reife LPL angegebenen Nukleotidsequenz hybridisiert, aufweist.

4. Vektor nach Anspruch 3, dadurch **gekennzeichnet,** daß der Vektor ein Plasmid ist.

5. Vektor nach Anspruch 4, dadurch **gekennzeichnet,** daß der Vektor das Plasmid pKC3, pKC9 oder pKC12 ist.

6. Vektor nach Anspruch 3, dadurch **gekennzeichnet,** daß der Vektor ein Phage oder ein Cosmid ist.

7. Vektor nach mindestens einem der Ansprüche 3 bis 6, dadurch **gekennzeichnet,** daß die DNA-Sequenzen nach b), die für einen Promotor codieren, ausgewählt sind aus der Gruppe TAKA-Amylase A-Promotor, gpdA-Promotor aus Aspergillus nidulans, Pektinesterase-Promotor aus Aspergillus niger, Polygalakturonidase-Promotor aus Aspergillus niger, Glucoamylase-Promotor aus Aspergillus niger oder Aspergillus awamori, Xylanase-Promotor aus Aspergillus foetidus, Lysophospholipase-Promotor aus Aspergillus foetidus, Cellobiohydrolase(cbh I)-Promotor aus Trichoderma reesei.

8. Vektor nach mindestens einem der Ansprüche 3 bis 7, dadurch **gekennzeichnet,** daß die DNA-Sequenzen nach b), die für eine Signalpeptidsequenz codieren, ausgewählt sind aus der Gruppe TAKA-Amylase A-Signalpeptidsequenz, Pektinesterase-Signalpeptidsequenz aus Aspergillus niger, Polygalakturonidase-Signalpeptidsequenz aus Aspergillus niger, Glucoamylase-Signalpeptidsequenz aus Aspergillus niger oder Aspergillus awamori, Xylanase-Signalpeptidsequenz aus Aspergillus foetidus, Lysophospholipase-Signalpeptidsequenz aus Aspergillus foetidus, Cellobiohydrolase-Signalpeptidsequenz (cbh I) aus Trichoderma reesei.

9. Transformierter Wirtsorganismus zur Herstellung von Lysophospholipase, dadurch **gekennzeichnet,** daß der Wirtsorganismus ein Aspergillus-Stamm oder ein Trichoderma reesei-Stamm ist und mit einem Vektor nach einem der Ansprüche 3 bis 8 transformiert ist.

10. Transformierter Wirtsorganismus nach Anspruch 9, dadurch **gekennzeichnet,** daß der Wirtsorganismus ein Stamm von Aspergillus niger, Aspergillus awamori, Aspergillus foetidus, Aspergillus japonicus, Aspergillus phoenicis, Aspergillus oryzae, Aspergillus sojae oder Trichoderma reesei ist.

11. Transformierter Wirtsorganismus nach Anspruch 10, dadurch **gekennzeichnet,** daß der Wirtsorganismus Aspergillus niger ATCC 10864, Aspergillus awamori NRRL 3112, Aspergillus awamori ATCC 11360, Aspergillus foetidus RH 3046, Aspergillus foetidus ATCC 11359, Aspergillus japonicus ATCC 16873, Aspergillus phoenicis CBS 136.52, Aspergillus oryzae NRRL 695, Aspergillus sojae RH3782 oder Trichoderma reesei ATCC 26921 ist.

12. Verfahren zur Herstellung von Lysophospholipase durch Fermentation eines transformierten Wirtsorganismus in einem geeigneten Kulturmedium und Gewinnung der Lysophospholipase aus dem zellfreien Kulturfiltrat, dadurch **gekennzeichnet,** daß der transformierte Wirtsorganismus ein Aspergillus-Stamm oder ein Trichoderma reesei-Stamm ist und mit einem Vektor nach einem der Ansprüche 3 bis 8 transformiert worden ist.

13. Verfahren nach Anspruch 12, dadurch **gekennzeichnet,** daß der transformierte Wirtsorganismus ein Stamm von Aspergillus niger, Aspergillus awamori, Aspergillus foetidus, Aspergillus japonicus, Aspergillus phoenicis, Aspergillus oryzae, Aspergillus sojae oder Trichoderma reesei ist.

14. Verfahren nach Anspruch 12 oder 13, dadurch **gekennzeichnet,** daß der transformierte Wirtsorganismus eine Transformante von Aspergillus niger ATCC 10864, Aspergillus awamori NRRL 3112, Aspergillus awamori ATCC 11360, Aspergillus foetidus RH 3046, Aspergillus foetidus ATCC 11359, Aspergillus japonicus ATCC 16873, Aspergillus phoenicis CBS 136.52, Aspergillus oryzae NRRL 695, Aspergillus sojae RH3782 oder Trichoderma reesei ATCC 26921 ist.

15. Enzymprodukt zur Verbesserung der Filtrationsleistung von Stärkehydrolysaten, dadurch **gekennzeichnet,** daß es eine mittels eines rekombinanten Aspergillus- oder T. reesei-Stammes hergestellte Lysophospholipase enthält.

16. Enzymprodukt nach Anspruch 15, dadurch **gekennzeichnet,** daß die Lysophospholipase eine aus Aspergillus foetidus isolierbare rekombinante Desoxyribonukleinsäure (DNA) mit der in SEQ ID NO 1 für die reife LPL angegebenen Nukleotidsequenz oder einer davon abgeleiteten Nukleotidsequenz, die unter stringenten Bedingungen mit der in SEQ ID NO 1 für die reife LPL angegebenen Nukleotidsequenz hybridisiert, ist.

17. Maltosesirup-Filtrat, dadurch **gekennzeichnet,** daß es mit einem Enzymprodukt nach Anspruch 15 oder 16 erhalten wurde.

Figur 1: Plasmidkarte von pKC9

Figur 2: Plasmidkarte von pKC3

Figur 3: Plasmidkarte von pKC12